# EUROPÄISCHE PATENTSCHRIFT

(11) **EP 1 758 980 B1**
(45) Veröffentlichungstag und Bekanntmachung des Hinweises auf die Patenterteilung: **29.12.2010**
(21) Anmeldenummer: 05753208.7
(22) Anmeldetag: 17.06.2005
(51) Int. Cl.: C12M 1/00

(54) **BIOREAKTOR**
BIOREACTOR
BIOREACTEUR

(30) Priorität: 23.06.2004 DE 102004030378
(43) Veröffentlichungstag der Anmeldung: 07.03.2007
(73) Patentinhaber: National Prawn Company, Al Lith 21961 (SA)
(72) Erfinder: KROON, Bernd, 27607 Langen (DE)
(74) Vertreter: Cohausz, Helge B.
(86) Internationale Anmeldenummer: PCT/EP2005/006537
(87) Internationale Veröffentlichungsnummer: WO 2006/000341

(56) Entgegenhaltungen:
- EP-A- 0 576 870
- GB-A- 2 344 651
- LAING I.: LABORATORY LEAFLET MAFF DIRECT. FISH-RCS., Nr. 67, 1991, Seiten 1-31, XP002347131 LOWESTOFT in der Anmeldung erwähnt

## Beschreibung

Die Erfindung betrifft ein Verfahren zur Herstellung eines Bioreaktors und eine Vorrichtung zum Ausüben des Verfahrens.

Mikroalgen finden in mehreren Bereichen Anwendung, z. B. als Futter für Fische im Larvalstadium, als Zusatzstoff für tierische oder humane Nahrungsmittel oder als Quelle für besondere Chemikalien aus dem Inhalt der Mikroalgenzellen. Einzellige Mikroalgen können nicht aus ihrer Umgebung unter wirtschaftlichen Gesichtspunkten gewonnen werden. Ein Produktionssystem ist notwendig, welches eine kontrollierte und sichere Produktion unter wirtschaftlichen Aspekten ermöglicht. Derzeit unterscheidet man zwischen offenen und geschlossenen Systemen bei der Mikroalgenproduktion. Offene Systeme sind Teich- oder Kanalanlagen, in welchem die Biomasse anwachsen soll. Nachteile: Keine Kontrollierbarkeit, Infektionsrisiko. Geschlossene Systeme stehen nicht in direktem Kontakt mit der Umwelt. Glasreaktoren, röhrenförmig oder zylindrisch, finden sowohl im Labor- wie auch im Industriebereich und in der Biotechnologie Anwendung. Ihr Nachteil ist, dass sie kostenintensiv, pflegeintensiv und bruchanfällig sind sowie während der Reinigung Produktionsausfälle anfallen. Acrylreaktoren, zylindrisch oder aus bearbeiteten Doppelstegplatten, haben bis auf die Bruchanfälligkeit dieselben Nachteile.

PE-Schlauchfolien werden in V-Formation verwendet, wobei das V die Unterseite bildet und die beiden oberen Enden der Schlauchfolie an einer Stellage befestigt werden. Mit Wasser befüllt ruht die Mitte des V's auf einer Boden- oder anderen Fläche. Die Belüftung stellt einen von oben eingeführten Luftschlauch dar. Das Befüllen geschieht von oben durch ein einfaches Loch in der Folie, das Ablassen des Inhalts durch ein unten am V eingestecktes Rohr, wobei der Anschluss nicht wasserdicht abgedichtet ist.

Die Nachteile dieser Technik:
- Leckagen an der V-Unterseite durch ungleichmäßige Belastung der Folie und an den Anschlüssen z. B. für Luftzufuhr, Zu- und Ablauf, welche einfach in die Folie eingesteckt sind.
- In der Nähe der V-Zone befinden sich Zonen, in denen die Flüssigkeit nicht gleichmäßig gemischt werden kann. Diese Stellen führen zu unerwünschten Ansammlungen von Biomasse an diesem Ort.
- Die V-Folien haben mit Wasser gefüllt ein hohes eigenes Gewicht, so dass eine den Kräften entsprechende Aufhängevorrichtung bzw. Stellage erforderlich ist. Anderenfalls kippt die Foliensäule um und reißt.
- Eine Produktion unter sterilen Bedingungen ist nicht möglich. Durch einen von oben durch ein einfaches Loch eingeführten Luftschlauch und unten durch den nicht wasserdichten Ablauf erhöht sich die Infektionsgefahr des PE-Folieninhaltes.

Die Veröffentlichung über eine Biomassenproduktion in einer PE-Schlauchfolie in V-Form ist im Jahre 1991 erschienen (I. Laing, Lab. Leafl. MAFF Direct. Fish-Rcs., Lowestoft, (67): 31 pp).

Aufgabe der Erfindung ist es, einen Bioreaktor zu schaffen, der bei einfacher Herstellung und Benutzung eine gleichmäßige Innenwand insbesondere am oberen und/oder unteren Ende schafft, frei von Leckagen ist und eine sterile Produktion ermöglicht.

Diese Aufgabe wird durch das erfindungsgemäße Verfahren mit einem beidseitig geschlossenen Folienschlauch mit folgenden Merkmalen gelöst:
a) Beim unteren, noch offenen Ende des Folienschlauches wird die Folienwand in mindestens drei vorzugsweise vier etwa gleich große Falten geformt,
b) danach werden alle Falten zu einem Stapel aufeinander gelegt,
c) die zwei seitlichen Längsbereiche des Faltenstapels werden nach innen derart umgebogen, dass die beiden Seitenränder auf die Längsmitte zu liegen und gegeneinander gerichtet sind,
d) die nun neu entstandenen zwei seitlichen Längsbereiche werden dann derart aufeinander geklappt, dass die Seitenränder innen liegen.

Auch wird eine Lösung dadurch geschaffen, dass eine Polyethylen (PE), PE-haltige oder eine geeignete Schlauchfolie mit einer Wandstärke von 50 bis 400 Mikrometer und einer Breite von mehr als 2 cm durch Luftüberdruck wie ein Rohr aufgestellt wird, wobei
a) die Folie zu vier gleichen Falten gezogen wird, wobei die offenen Seiten der Falten zum Mittelpunkt der Querschnittsfläche zeigen und drei der Falten zu einer Seite zusammengelegt werden, über die die letzte Falte gelegt wird, die dann gesamt hälftig mittig gefaltet werden, und die abhängig von der Folienbreite beliebig oft hälftig mittig wiederholt gefaltet werden können, woraufhin die zusammengedrückten Falten bei 250 - 300 °C verschmolzen werden und
b) wasser- und luftdichte Anschlüsse eingearbeitet werden durch Dehnung der Folie mittels eines kegelförmigen Werkzeugs und anschließenden Stechens eines Loches in die Folie und anschließendem Einsetzen des erforderlichen Formteils, so dass dieses von der Folienlippe von innen umschlossen wird, wobei das Formteil durch Umstülpen eines Schlauchstücks entsprechenden Durchmessers mit aufgesetztem Diffuser am anderen Ende über das Formteil mit der sich anschmiegenden Folienlippe das Formteil eine eigene Dichtung erhält.

Vorteilhafte Ausgestaltungen sind in den Unteransprüchen aufgeführt.

Unter anderem werden folgende Vorteile erzielt:
1. Sterile Produktionsbedingungen im Innenraum des PE-Folienreaktors und Verhinderung von Leckagen an den Anschlüssen durch wasser- und luftdichte in die Folie eingearbeitete Anschlüsse: Luftzufuhr-, bei Bedarf CO₂-, Zulauf- und Ablaufanschlüsse. Eine Schweißnaht oben und eine Schmelzung des Bodens des PE-Folienreaktors erzeugen einen geschlossenen Innenraum ohne Infektionsrisiko.
2. Es entstehen keine Leckagen oder Risse am Boden des PE-Folienreaktors. Durch eine zentrale Faltung des Folienendes der späteren Unterseite der Foliensäule mit anschließender Einschmelzung dieses Außenendes erhält die Unterseite der Foliensäule unter Luftdruck und mit Wasser befüllt eine zentrisch konische Form und damit bei Aufliegen auf einem Tisch oder Sockel einen gleichmäßigen runden Boden, wobei die Gewichtsverteilung bzw. Kräfte der Wassersäule nach unten gleichmäßig wirken können.
3. Es ist keine aufwendige Stellage für die Befestigung notwendig. Durch die zentrierte Schmelzung des Bodens des PE-Folienreaktors und durch den eingebrachten Überdruck steht er nahezu frei. Es ist lediglich zur Fixierung eine leichte Befestigung der Oberseite des PE-Folienreaktors notwendig.
4. Kostengünstige PE-Folien und PE-Schläuche der Anschlüsse des Reaktors können nach mehrmaligem Gebrauch als PE-Rohstoff recycelt werden. Belüfter und Anschlüsse können auch leicht entfernt werden und nach einer Reinigung wieder für neue PE-Folienreaktoren Verwendung finden.
5. Eine Reinigung ist nicht erforderlich und damit entfallen Produktionsausfallzeiten. Die PE-Folienreaktoren können auf Vorrat gefertigt werden.
6. Eine schnelle Produktionserweiterung ist möglich. Die Erweiterung erfolgt einfach über eine Reihenanordnung der PE-Folienreaktoren, wobei Zu- und Ablaufanschlüsse jeweils in ein gemeinsames Hauptrohr münden. So stellt jeder PE-Folienreaktor ein Modul der Gesamtproduktionsanlage dar.
7. Durch die Nutzung des PE-Folienreaktors als stehendes Rohr entstehen keine unerwünschten Biomassenablagerungen.

Ein Ausführungsbeispiel der Erfindung ist in den Zeichnungen dargestellt und wird im Folgenden näher beschrieben. Es zeigen
- Bild I: ein Erstellen von vier ersten Falten eines Folienendes,
- Bild II: das Legen dreier Falten zu einer Seite und das Legen der vierten Falte über die übrigen,
- Bild III: ein erstmaliges hälftiges Falten des entstandenen Faltenpaketes,
- Bild IV: ein zweites hälftiges Falten des neu entstandenen Faltenpaketes, und
- Bild V: das Einsetzen eines Druckventils.

Die Erfindung ist ein PE-Folienreaktor für biotechnologische Produktions- und Verarbeitungsprozesse. Es ist ein unter Druck gebrachter Polyethylen (PE)-Folienreaktor zur Produktion von marinen Mikroalgen. Ferner geht es um die Herstellung von PE-Folienreaktoren.

Er dient in der Biotechnologie zur kontrollierten und sicheren Produktion pflanzlicher Rohstoffe wie Mikroalgen und Makroalgen, zur Zucht von photoautotrophen und nicht phototrophen Mikroorganismen wie Bakterien, Hefearten und Hefepilzen, zur Aufzucht von Zooplankton, zur Umwandlung bzw. Verwertung von Nitraten, Phosphaten und CO₂ im Rahmen eines Wachstumsprozesses von nutzbarer Biomasse.

Für jegliche Anwendungen, wo Partikel biologischer oder nicht biologischer Herkunft in gleichzeitigen Kontakt mit Gas, rein oder als Mischgas und/oder Luft, und/oder Lichte (Kunst- oder natürliches Licht) und/oder Flüssigkeit gebracht werden müssen, ist der PE-Folienreaktor anwendbar.

Außerhalb der genannten Anwendungen kann der Folienreaktor als Behältnis für Flüssigkeiten genutzt werden.

Ein PE-Folienreaktor wird als einzelne Foliensäule mit wasserdichten Anschlüssen für Luft- bzw. Gaszufuhr und Zu- und Ablauf gefertigt und unter Luftdruck wie ein stehendes Rohr genutzt. Er ist oben mit einer Schweißnaht verschlossen und die Unterseite ist, nachdem das Ende so gefaltet wurde, dass alle Falten mit der offenen Seite zentral zum Mittelpunkt der Querschnittsfläche führen, durch eine spezielle Schmelztechnik wasserdicht verschlossen. Der PE-Folienreaktor kann durch den Luftüberdruck wie ein Rohr aufgestellt werden und ruht gleichmäßig auf seiner Querschnittsfläche. Die Belüftung oder Begasung des Produktionsinnenraumes erfolgt über einen wasserdicht eingesetzten Diffuser über dem Boden des PE-Folienreaktors. Die Wandstärke des PE-Folienreaktors liegt zwischen 50 und 400 Mikrometer. Die Breite der PE-Folie beträgt > 2 cm.

Die Erfindung ist u. a. gekennzeichnet durch das Unterdrucksetzen des PE-Folienreaktors, sodass dieser nahezu selbst stehend wird und nur noch zur Fixierung eine leichte Befestigung benötigt.

Eine weitere Erfindung ist das Verschmelzen des Bodens des PE-Folienreaktors. Zuvor wird eine spezielle Falttechnik angewandt, derart, dass die Folie zu vier gleichen Falten gezogen wird (Bild I a1 bis a4), wobei die offenen Seiten der Falten zum Mittelpunkt der Querschnittsfläche zeigen (Bild I b1 bis b4). Danach werden die drei Falten außer der linken (Bild II a4) nach rechts zusammengelegt (Bild II a1 bis a3) und dann die linke vierte Falte ebenfalls über die drei bereits zusammengelegten Faltungen gelegt (Bild II). Alle Falten liegen jetzt aufeinander und werden hälftig zweimal hintereinander zueinander aufeinander gefaltet (Bild III + IV). Zum Abschluss werden die Faltenseiten noch einmal zusammengepresst. Die eng zusammengefalteten Folienschichten bilden jetzt ein stangenförmiges Gebilde, in welchem die Folienschichten ohne Lufteinschlüsse zusammengepresst worden sind.

Als Hilfsmittel kann ein Band die Folienschichten der "Folienstange" zusammenhalten. Jetzt erfolgt die Verschmelzung an einer 250 bis 300 Grad Celsius heißen Teflonschicht, welches zu einer einige Millimeter dicken Verschmelzung der "Folienstange" führt. Bedingt durch die vorherige Falttechnik liegt die Verschmelzung zentral im Mittelpunkt der Querschnittsfläche des PE-Folienreaktors, Abb. I-IV. Wenn der PE-Folienreaktor unter Luftdruck gesetzt wird, stülpt sich der Boden konisch und gleichmäßig zentrisch gefaltet rund aus. Diese Technik verhindert Risse oder ungleichmäßige Belastungen am Boden des PE-Folienreaktors. Weiterhin ermöglicht sie eine gleichmäßige Belüftung des Reaktorinhaltes durch den eingearbeiteten Diffuser.

Weiterhin wird die vorliegende Erfindung durch eine Technik gekennzeichnet, die es ermöglicht, in PE-Folien wasser- bzw. luftdichte Anschlüsse einzuarbeiten. Die Technik besteht darin, die PE-Folie mit einem kegelförmigen Werkzeug zuerst zu dehnen und dann ein Loch zu stechen. Es wird kein PE-Material entfernt. Um das nun entstandene Loch hat sich eine kreisrunde Folienlippe gebildet. Danach kann das Formteil z. B. ein Druckventil (Bild V d) in die PE-Folie (Bild V a) eingesetzt werden. Die Folienlippe umschließt das Formteil von innen. Mit einem Schlauchstück entsprechenden Durchmesser mit aufgesetztem Diffuser am anderen Ende (Bild V b), welches über das Formteil mit der sich anschmiegenden Folienlippe gestülpt wird, erhält das Formteil eine eigene Dichtung, die wasserdicht ist und dem Druck in dem PE-Säulenreaktor standhält. Jetzt kann z. B. eine Luft- oder Gaszufuhr z. B. CO₂ (Bild V c) an der Außenseite des Folienreakors am Druckventil angeschlossen werden. Alle weiteren Anschlüsse wie Zu- und Abfuhranschlüsse für den PE-Reaktorinhalt werden in gleicher Weise gefertigt.

Der PE-Folienreaktor kann als Kreislaufanlage zur Zucht von marinen Mikroalgen verwendet werden. Eine oder mehrere PE-Folienreaktoren (Folienbreite 20 cm, Folienstärke 200 Mikron, Länge 2 Meter, Füllvolumen 25 Liter pro PE-Folienreaktor bei einem Reaktordurchmesser im gefüllten Zustand von 12,7 cm werden hintereinander angeordnet, sodass die unteren Enden der Folien direkt über einem Sockel oder Tisch hängen. Danach wird an dem dafür vorgesehenen Anschluss Luft in die Folie eingebracht (Überdruck bis 250 Millibar), bis sie prall ist. Danach füllt man die Säule an dem entsprechenden Zulauf mit z. B. Meerwasser und Nährstoffen und einer Impfkultur einer Mikroalgenart auf. Ist die Kultur nach einigen Tagen angewachsen, kann der Inhalt teilweise (oder ganz) über den Ablauf bzw. Hauptablaufrohr abgelassen werden und die Mikroalgenzellen mittels gewünschter Abtrenntechnik (Zentrifuge, Cross-Flow oder andere Methoden) vom umliegenden Meerwasser (Medium) getrennt werden. Das so gewonnene Medium kann wieder, mit Nährstoffen angereichert, in die PE-Folienreaktoren zurückgeführt werden, sodass z. B. bei einer Teilentleerung die Restmenge wieder mit dem zugeführten Medium anwachsen kann.

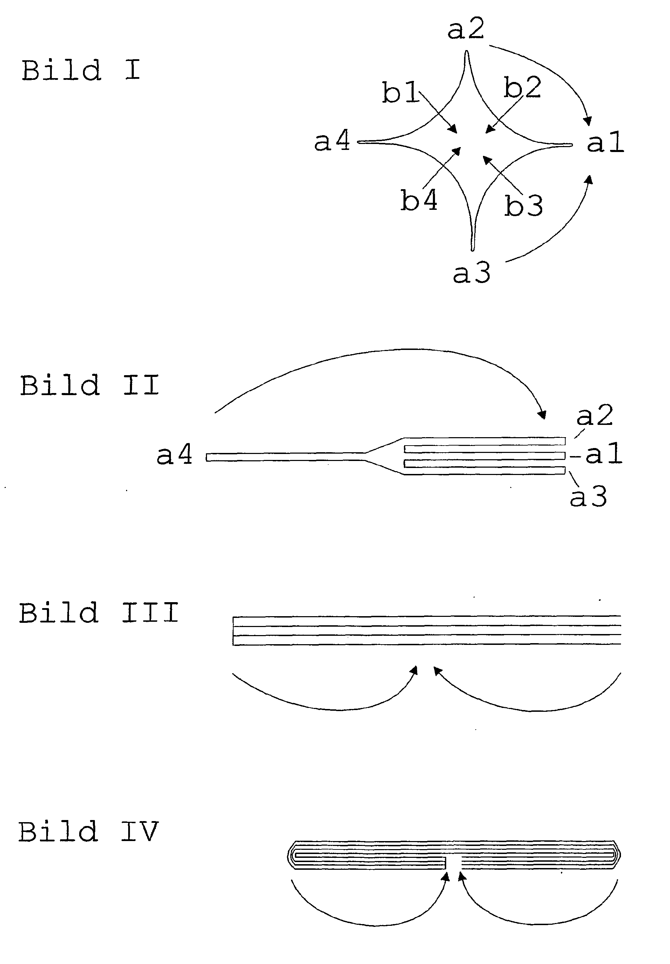

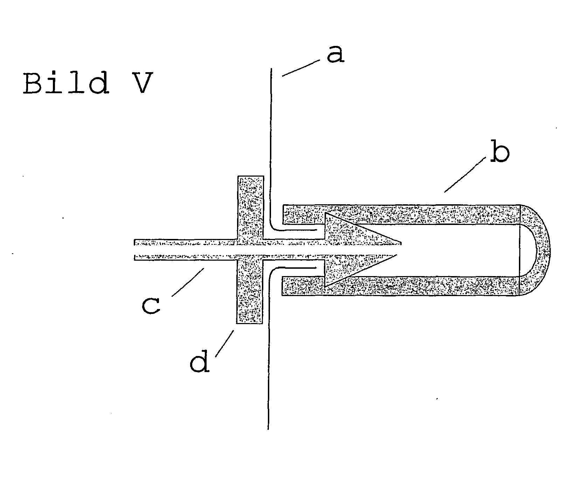

## Patentansprüche

1. Verfahren zur Herstellung eines Bioreaktors, **gekennzeichnet durch** einen beidseitig geschlossenen Folienschlauch mit folgenden Merkmalen:
a) Beim unteren, noch offenen Ende des Folienschlauches wird die Folienwand in mindestens drei vorzugsweise vier etwa gleich große Falten geformt,
b) danach werden alle Falten zu einem Stapel aufeinander gelegt,
c) die zwei seitlichen Längsbereiche des Faltenstapels werden nach innen derart umgebogen, dass die beiden Seitenränder auf die Längsmitte zu liegen und gegeneinander gerichtet sind,
d) die nun neu entstandenen zwei seitlichen Längsbereiche werden dann derart aufeinander geklappt, dass die Seitenränder innen liegen.

2. Verfahren zur axenischen oder nicht axenischen Herstellung oder Massenproduktion oder Zucht von tierischen oder pflanzlichen Organismen oder Zellen davon o.a. Biomasse in gleichzeitigem Kontakt mit den für die Zucht, Herstellung oder Produktion erforderlichen Stoffen in einem geschlossenen System, insbesondere nach Anspruch 1, **dadurch gekennzeichnet, dass** eine Polyethylen (PE), PE-haltige oder eine geeignete Schlauchfolie mit einer Wandstärke von 50 bis 400 Mikrometer und einer Breite von mehr als 2 cm durch Luftüberdruck wie ein Rohr aufgestellt wird, wobei
a) die Folie zu vier gleichen Falten gezogen wird, wobei die offenen Seiten der Falten zum Mittelpunkt der Querschnittsfläche zeigen und drei der Falten zu einer Seite zusammengelegt werden, über die die letzte Falte gelegt wird, die dann gesamt hälftig mittig gefaltet werden, und die abhängig von der Folienbreite beliebig oft hälftig mittig wiederholt gefaltet werden können, woraufhin die zusammengedrückten Falten bei 250 - 300 °C verschmolzen werden und
b) wasser- und luftdichte Anschlüsse eingearbeitet werden durch Dehnung der Folie mittels eines kegelförmigen Werkzeugs und anschließenden Stechens eines Loches in die Folie und anschließendem Einsetzen des erforderlichen Formteils, so dass dieses von der Folienlippe von innen umschlossen wird, wobei das Formteil durch Umstülpen eines Schlauchstücks entsprechenden Durchmessers mit aufgesetztem Diffuser am anderen Ende über das Formteil mit der sich anschmiegenden Folienlippe das Formteil eine eigene Dichtung erhält.

3. Verfahren nach Anspruch 1 oder 2, **dadurch gekennzeichnet, dass** vor dem Verschließen des unteren oder oberen Endes Zu- und/oder Abflüsse in die Folienwand nahe dem unteren oder oberen Ende eingebracht werden.

4. Verfahren nach Anspruch 3, **dadurch gekennzeichnet, dass** durch Einstechen und/oder Eindrehen eines Stechinstrumentes in die Folienwand ein vorgestülpter ringförmiger Wandbereich um die Öffnung entsteht, der von Teilen des Zu- und/oder Abflussteils eingeklemmt wird, nachdem dieses in die Öffnung eingedrückt worden ist.

5. Verfahren nach einem der vorherigen Ansprüche, **dadurch gekennzeichnet, dass** der Folienschlauch als Behältnis für Flüssigkeit verwendbar ist insbesondere zum Mischen und/oder in Kontaktbringen von Feststoffen, Gasen und Flüssigkeiten.

6. Verfahren nach einem der vorherigen Ansprüche, **dadurch gekennzeichnet, dass** im Folienschlauch nichtbiologische Partikel, in gleichzeitigem Kontakt mit Gas und/oder Luft und/oder Flüssigkeiten und/oder anderen Partikeln und/oder Licht bringbar sind.

7. Verfahren nach einem der vorherigen Ansprüche, **dadurch gekennzeichnet, dass** im Folienschlauch die Verwertung bzw. Umwandlung von Kohlendioxid, Nitraten oder Phosphaten oder anderen Stoffen im Rahmen eines Wachstumsprozesses von Biomasse erfolgt.

8. Verfahren nach einem der vorherigen Ansprüche, **dadurch gekennzeichnet, dass** Gase zur Gaswaschung in den Folienschlauch eingeleitet werden.

9. Verfahren nach einem der vorherigen Ansprüche, **dadurch gekennzeichnet, dass** zur Befestigung des Folienreaktors ein geringer Luftüberdruck und insbesondere kein stabiles Aufhängesystem verwendet wird.

10. Vorrichtung zum Ausüben des Verfahrens nach einem der vorherigen Ansprüche, **gekennzeichnet durch** einen beidseitig geschlossenen Folienschlauch, an dessen unterem, noch offenen Ende die Folienwand in mindestens drei vorzugsweise vier etwa gleich große Falten geformt ist, alle Falten zu einem Stapel aufeinander gelegt sind, die zwei seitlichen Längsbereiche des Faltenstapels nach innen derart umgebogen sind, dass die beiden Seitenränder auf der Längsmitte liegen und gegeneinander gerichtet sind, und die zwei seitlichen Längsbereiche derart aufeinander geklappt sind, dass die Seitenränder innen liegen.

## Claims

1. Method for manufacturing a bioreactor, **characterized by** a tubular film closed at both ends, having the following features:
a) At the lower, still open end of the tubular film the film wall is shaped into at least three, preferably four folds of approximately the same size,
b) then all folds are laid one upon another to form a stack,
c) the two lateral longitudinal regions of the fold stack are bent inward in such a manner that the two lateral edges lie along the central longitudinal axis and are oriented toward each other,
d) the now newly produced two lateral longitudinal regions are then laid one on top of the other so that the lateral edges lie inwards.

2. Method for axenic or non-axenic production or mass production or culturing of animal or vegetable organisms or cells thereof, or other biomass, in simultaneous contact with the substances required for culturing, manufacture or production in a closed system, in particular according to claim 1, **characterized in that** a polyethylene (PE), PE-containing or suitable tubular film with a wall thickness of 50 to 400 micrometers and a width of more than 2 cm is made to stand vertically like a tube by means of air overpressure, wherein
a) the film is drawn into four identical folds, with the open ends of the folds pointing to the center of the cross-sectional area, and three of the folds are laid together on one side, over which the last fold is laid, the totality of folds are then folded in half toward the center and can be repeatedly folded in half toward the center as many times as the width of the film will allow, after which the compressed folds are melted together at 250 - 300 °C, and
b) watertight and airtight connections are integrated by stretching the film with the aid of a conical tool and subsequently piercing a hole in the film and inserting the necessary molding so that the latter is surrounded on the inside by the film lip, the molding being given a seal of its own by pushing one end of a piece of tubing of appropriate diameter, which has an diffuser attached to its other end, over the molding and its conforming film lip.

3. A method according to claim 1 or 2, **characterized in that**, before closing the lower or upper end, inlets and/or outlets are integrated in the film wall near to the lower or upper end.

4. A method according to claim 3, **characterized in that,** by forcing and/or screwing a piercing instrument into the film wall, a protuberant annular wall zone is formed around the opening and is clamped by sections of the inlet and/or outlet part once the latter has been pressed into the opening.

5. A method according to any of the preceding claims, **characterized in that** the tubular film can be used as a recipient for liquid, in particular for mixing solids, gases and liquids and/or bringing these into contact with one another.

6. A method according to any of the preceding claims, **characterized in that** within the tubular film non-biological particles can brought into simultaneous contact with gas and/or air and/or liquids and/or other particles and/or light.

7. Method according to any of the preceding claims, **characterized in that** within the tubular film carbon dioxide, nitrates or phosphates or other substances are digested or converted in the context of a growth process of biomass.

8. A method according to any of the preceding claims, **characterized in that** gases for gas cleaning are introduced into the tubular film.

9. A method according to any of the preceding claims, **characterized in that** for fixing of the film reactor slight air overpressure is applied and in particular no rigid suspension system is used.

10. A device for executing the method according to any of the preceding claims **characterized by** a tubular film closed at both ends, at the lower, still open end of which the film wall is shaped into at least three, preferably four folds of approximately the same size, all folds are laid one upon another to form a stack, the two lateral longitudinal regions of the fold stack are bent inward in such a manner that the two lateral edges lie along the central longitudinal axis and are oriented toward each other, and two lateral longitudinal regions are then laid one on top of the other so that the lateral edges lie inwards.

## Revendications

1. Procédé de fabrication d'un bioréacteur, **caractérisé par** un film tubulaire fermé en ses deux extrémités, doté des caractéristiques suivantes :
a) En l'extrémité inférieure, encore ouverte, du film tubulaire, la paroi du film est formée pour donner au moins trois, de préférence quatre, plis de taille comparable,
b) ensuite tous les plis sont empilés les uns sur les autres,
c) les deux zones longitudinales latérales de l'empilage de plis sont repliées vers l'intérieur, de façon à refermer les deux bords latéraux sur le milieu dans le sens de la longueur et les orienter l'un vers l'autre,
d) les deux nouvelles zones longitudinales latérales ainsi formées sont ensuite rabattues l'une sur l'autre, de façon à placer les bords latéraux à l'intérieur.

2. Procédé de fabrication ou de production en masse ou de culture, axénique ou non axénique, d'organismes animaux ou végétaux ou de cellules de ceux-ci ou de toute autre biomasse en contact simultané avec les éléments nécessaires à la culture, à la fabrication ou à la production dans un système fermé, en particulier selon la revendication n° 1, **caractérisé en ce qu'**un film tubulaire en Polyéthylène (PE), contenant du PE ou appropriée, d'épaisseur de paroi de 50 à 400 micromètres et de largeur dépassant 2 cm, est dressé tel un tuyau par surpression de l'air, cependant que
a) le film est tiré pour former quatre plis identiques, cependant que les extrémités ouvertes des plis pointent vers le centre de la surface de la section et trois des plis sont rassemblés d'un côté, où vient se poser le dernier pli, et la totalité est ensuite pliée en deux en son milieu et peut, en fonction de la largeur du film, être pliée en deux en son milieu de façon répétée, autant de fois que souhaité, sur quoi les plis comprimés sont fondus ensemble à 250-300 °C et
b) des raccords, étanches à l'eau comme à l'air, sont intégrés en étirant le film à l'aide d'un outil conique, puis en perçant un trou dans le film et ensuite en insérant l'élément préformé requis, les lèvres du film se referment ainsi de l'intérieur sur lui, cependant qu'un bout de flexible de diamètre correspondant, doté d'un diffuseur en son autre extrémité, vient prendre sur les lèvres du film, qui épousent la forme de l'élément préformé, conférant à l'élément préformé une étanchéité propre.

3. Procédé selon la revendication n° 1 ou n° 2, **caractérisé en ce que** des admissions et/ou des évacuations sont intégrées dans la paroi du film, près de son extrémité inférieure ou supérieure, avant la fermeture de l'extrémité inférieure ou supérieure.

4. Procédé selon la revendication n° 3, **caractérisé en ce que** le piquage et/ou le vissage d'un instrument perçant dans la paroi du film provoque, autour de l'ouverture, une zone de cloison annulaire emboutie en avant, sur laquelle viennent prendre des parties de l'élément d'admission et/ou d'évacuation lorsque celui-ci est inséré dans l'ouverture.

5. Procédé selon une des revendications précédentes, **caractérisé en ce que** le film tubulaire peut être utilisé comme récipient pour liquide, en particulier pour mélanger et/ou mettre en contact des matières solides, des gaz et des liquides.

6. Procédé selon une des revendications précédentes, **caractérisé en ce que** des particules non biologiques peuvent être amenées dans le film tubulaire, tout en restant en contact avec du gaz et/ou de l'air et/ou des liquides et/ou d'autres particules et/ou de la lumière.

7. Procédé selon une des revendications précédentes, **caractérisé en ce que** dans le film tubulaire a lieu l'exploitation et/ou la transformation de dioxyde de carbone, de nitrates ou de phosphates ou d'autres matières dans le cadre du processus de croissance d'une biomasse.

8. Procédé selon une des revendications précédentes, **caractérisé en ce que** des gaz sont introduits dans le film tubulaire à des fins d'absorption-neutralisation.

9. Procédé selon une des revendications précédentes, **caractérisé en ce que** la fixation du réacteur à film est assurée par une faible surpression d'air et, en particulier, sans système de suspension stable.

10. Dispositif pour exercer le procédé selon une des revendications précédentes, **caractérisé par** un film tubulaire fermé en ses deux extrémités, en l'extrémité inférieure, encore ouverte, duquel la paroi en film forme au moins trois, de préférence quatre, plis de taille comparable, tous les plis sont ensuite empilés les uns sur les autres, les deux zones longitudinales latérales de l'empilage de plis sont repliées vers l'intérieur, de façon à refermer les deux bords latéraux sur le milieu dans le sens de la longueur et les orienter l'un vers l'autre, et les deux zones longitudinales latérales sont rabattues l'une sur l'autre, de façon à placer les bords latéraux à l'intérieur.
